(19) Europäisches Patentamt
European Patent Office
Office européen des brevets

(11) Veröffentlichungsnummer: **0 479 055 A1**

(12) **EUROPÄISCHE PATENTANMELDUNG**

(21) Anmeldenummer: **91115908.5**

(22) Anmeldetag: **19.09.91**

(51) Int. Cl.[5]: **C07D 239/60**, C07D 239/52, C07D 251/30, C07D 403/12, C07C 251/32, A01N 43/54, A01N 43/66

(30) Priorität: **29.09.90 DE 4030929**

(43) Veröffentlichungstag der Anmeldung: **08.04.92 Patentblatt 92/15**

(84) Benannte Vertragsstaaten: **AT BE CH DE ES FR GB IT LI NL SE**

(71) Anmelder: **BASF Aktiengesellschaft**
**Carl-Bosch-Strasse 38**
**W-6700 Ludwigshafen(DE)**

(72) Erfinder: **Rheinheimer, Joachim, Dr.**
**Merziger Strasse 24**
**W-6700 Ludwigshafen(DE)**
Erfinder: **Eicken, Karl, Dr.**
**Am Huettenwingert 12**
**W-6706 Wachenheim(DE)**
Erfinder: **Vogelbacher, Uwe Josef, Dr.**
**Niedererdstrasse 56**
**W-6700 Ludwigshafen(DE)**
Erfinder: **Westphalen, Karl-Otto, Dr.**
**Mausbergweg 58**
**W-6720 Speyer(DE)**
Erfinder: **Gerber, Matthias, Dr.**
**Ritterstrasse 3**
**W-6704 Mutterstadt(DE)**
Erfinder: **Walter, Helmut, Dr.**
**Gruenstadter Strasse 82**
**W-6719 Obrigheim(DE)**

(54) **Benzaldoximetherderivate.**

(57) Benzaldoximetherderivate der Formel I

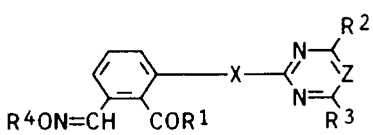

I

($R^1$ Succiniminoxy; unsubstituiertes oder substituiertes Hetaryl; $OR^5$, in der $R^5$ substituiertes oder unsubstituiertes Cycloalkyl, Alkyl, Alkenyl, Alkinyl, Phenyl oder eine Oximgruppe bedeutet; $OR^8$, in der $R^8$ Wasserstoff, Alkalimetall-, Erdalkalimetallkation, Ammonium oder organisches Ammonium bedeutet;

$R^2$, $R^3$ $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylamino, Di-($C_1$-$C_4$-alkyl)amino und/oder $C_1$-$C_4$-Alkylthio;

$R^4$ $C_1$-$C_8$-Alkyl, $C_3$-$C_6$-Alkenyl, $C_3$-$C_6$-Halogenalkenyl, unsubstituiertes oder substituiertes Benzyl;

X Sauerstoff, Schwefel;

Z Stickstoff, Methingruppe)

ferner die Herstellung dieser Stoffe, Zwischenprodukte dafür, und die Verwendung der Verbindungen I als Herbizide und Bioregulatoren.

EP 0 479 055 A1

Die vorliegende Erfindung betrifft Benzaldoximetherderivate der Formel I

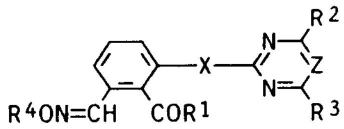

I

in der die Substituenten folgende Bedeutung haben:

$R^1$ Succinimidoxy;
einen 5-gliedrigen Heteroaromaten, enthaltend zwei oder drei Stickstoffatome, welcher ein bis zwei Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
einen Rest -$OR^5$, in dem

$R^5$ eine $C_3$-$C_{12}$-Cycloalkylgruppe, welche ein bis drei $C_1$-$C_4$-Alkylreste tragen kann;
eine $C_1$-$C_{10}$-Alkylgruppe, welche ein bis fünf Halogenatome und/oder einen der folgenden Reste tragen kann:
$C_1$-$C_4$-Alkoxy, $C_1$-$C_4$-Alkylthio, Cyano, $C_1$-$C_8$-Alkylcarbonyl, $C_3$-$C_{12}$-Cycloalkyl, $C_1$-$C_8$-Alkoxycarbonyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
eine $C_2$-$C_6$-Alkylgruppe, welche in der 2-Position einen der folgenden Reste trägt: $C_1$-$C_6$-Alkoxyimino, $C_3$-$C_6$-Alkenyloxyimino, $C_3$-$C_6$-Halogenalkenyloxyimino oder Benzyloxyimino;
eine $C_3$-$C_6$-Alkenyl- oder eine $C_3$-$C_6$-Alkinylgruppe, wobei diese Gruppen ihrerseits ein bis fünf Halogenatome tragen können;
unsubstituiertes oder ein- bis dreifach durch $C_1$-$C_4$-Alkyl oder $C_1$-$C_4$-Alkoxy substituiertes oder ein- bis fünffach durch Halogen substituiertes Phenyl; oder
den Rest -$N=CR^6R^7$, in dem
$R^6$ und $R^7$ $C_1$-$C_{20}$-Alkyl, welches einen Phenylrest, eine $C_1$-$C_4$-Alkoxy- und/oder eine $C_1$-$C_4$-Alkylthiogruppe tragen kann, Phenyl oder gemeinsam eine $C_3$-$C_{12}$-Alkylenkette, welche ein bis drei $C_1$-$C_3$-Alkylgruppen tragen kann, bedeuten;
bedeutet, oder
einen Rest -$OR^8$, in dem

$R^8$ für Wasserstoff, ein Alkalimetallkation, das Äquivalent eines Erdalkalimetallkations, Ammonium oder ein organisches Ammoniumion steht;

$R^2$, $R^3$ $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylamino, Di-($C_1$-$C_4$-alkyl)-amino und/oder $C_1$-$C_4$-Alkylthio;

$R^4$ $C_1$-$C_8$-Alkyl, $C_3$-$C_6$-Alkenyl, $C_3$-$C_6$-Halogenalkenyl oder Benzyl, welches im Phenylring ein bis drei der folgenden Substituenten tragen kann: $C_1$-$C_3$-Alkyl, Halogen, $C_1$-$C_4$-Alkoxy;

X Sauerstoff oder Schwefel;

Z Stickstoff oder eine Methingruppe =CH-.

Weiterhin betrifft die Erfindung Verfahren zur Herstellung dieser Verbindungen, ihre Verwendung als Herbizide und Wachstumsregulatoren sowie Verbindungen der Formel IV

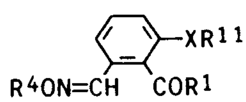

IV

als Zwischenprodukte zur Herstellung der Verbindungen I, in der $R^{11}$ Wasserstoff, Acetyl oder Methyl bedeutet.

In der Literatur (EP-A 223 406, EP-A 287 072, EP-A 287 079, EP-A 249 708 und EP-A 360 163) sind herbizid wirksame aromatische Carbonsäurederivate beschrieben. Sie enthalten jedoch keine Oximethergruppen und ihre herbizide Wirkung ist unbefriedigend.

Der Erfindung lagen weitere, besonders wirksame Verbindungen mit verbesserten herbiziden Eigenschaften sowie mit pflanzenwachstumsregulierenden Eigenschaften als Aufgabe zugrunde.

Demgemäß wurden die eingangs definierten Verbindungen I gefunden.

Außerdem wurden Verfahren zur Herstellung der Verbindungen I, Zwischenprodukte der Formel IV, die

Verbindung I enthaltende herbizide Mittel, Verfahren zur Bekämpfung unerwünschten Pflanzenwuchses, die Verwendung der Verbindungen I als Herbizide, Mittel zur Beeinflussung sowie Verfahren zur Regulierung des Pflanzenwuchses gefunden.

Im Hinblick auf die bestimmungsgemäße Verwendung der Benzaldoximetherderivate I als Herbizide und Wachstumsregulatoren kommen als Substituenten vorzugsweise folgende Reste in Betracht:

$R^1$ Succinimidoxy;

5-gliedriges Heteroaryl wie Pyrrolyl, Pyrazolyl, Imidazolyl und Triazolyl, insbesondere Imidazolyl und Pyrazolyl, wobei der aromatische Rest über Stickstoff gebunden ist und seinerseits ein bis zwei Halogenatome, insbesondere Fluor und Chlor und/oder ein bis zwei der folgenden Reste tragen kann:

$C_1$-$C_4$-Alkyl, wie Methyl, Ethyl, Propyl, 1-Methylethyl, Butyl, 1-Methylpropyl, 2-Methylpropyl und 1,1-Dimethylethyl, vorzugsweise Methyl, Ethyl und 1-Methylethyl;

$C_1$-$C_4$-Halogenalkyl, vorzugsweise $C_1$-$C_2$-Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifluormethyl, 2,2,2-Trifluorethyl und Pentafluorethyl;

$C_1$-$C_4$-Alkoxy wie Methoxy, Ethoxy, Propoxy, 1-Methylethoxy, Butoxy;

$C_1$-$C_4$-Halogenalkoxy, insbesondere $C_1$-$C_2$-Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und/oder

$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio;

einen Rest $-OR^5$, in dem $R^5$ vorzugsweise folgende Bedeutung hat:

$R^5$ $C_3$-$C_6$-Cycloalkyl wie Cyclopropyl, Cyclobutyl, Cyclopentyl und Cyclohexyl, welches ein bis drei $C_1$-$C_4$-Alkylreste, insbesondere Methyl und Ethyl, tragen kann;

$C_1$-$C_{10}$-Alkyl wie Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl, Octyl, Nonyl und Decyl, vorzugsweise $C_1$-$C_6$-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, welches für $C_1$ ein bis drei, und für $C_2$-$C_{10}$ ein bis fünf Halogenatome, insbesondere Fluor und Chlor und/oder einen der folgenden Reste tragen kann:

$C_1$-$C_4$-Alkoxy, insbesondere Methoxy, Ethoxy und 1-Methylethoxy;

$C_1$-$C_4$-Alkylthio, insbesondere Methylthio und Ethylthio; Cyano;

$C_1$-$C_6$-Alkylcarbonyl wie Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl;

$C_3$-$C_6$-Cycloalkyl wie Cyclopropyl, Cyclopentyl und Cyclohexyl;

$C_1$-$C_8$-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl, n-Pentyloxycarbonyl, 1-Methylbutyloxycarbonyl, 2-Methylbutyloxycarbonyl, 3-Methylbutyloxycarbonyl, 1,2-Dimethylpropyloxycarbonyl, 1,1-Dimethylpropyloxycarbonyl, 2,2-Dimethylpropyloxycarbonyl, 1-Ethylpropyloxycarbonyl, n-Hexyloxycarbonyl, 1-Methylpentyloxycarbonyl, 2-Methylpentyloxycarbonyl, 3-Methylpentyloxycarbonyl, 4-Methylpentyloxycarbonyl, 1,2-Dimethylbutyloxycarbonyl, 1,3-Dimethylbutyloxycarbonyl, 2,3-Dimethylbutyloxycarbonyl, 1,1-Dimethylbutyloxycarbonyl, 2,2-Dimethylbutyloxycarbonyl, 3,3-Dimethylbutyloxycarbonyl, 1,1,2-Trimethylpropyloxycarbonyl, 1,2,2-Trimethylpropyloxycarbonyl, 1-Ethylbutyloxycarbonyl, 2-Ethylbutyloxycarbonyl, 1-Ethyl-2-methylpropyloxycarbonyl, n-Heptyloxycarbonyl, 1-Methylhexyloxycarbonyl, 2-Methylhexyloxycarbonyl, 3-Methylhexyloxycarbonyl, 4-Methylhexyloxycarbonyl, 5-Methylhexyloxycarbonyl, 1-Ethylpentyloxycarbonyl, 2-Ethylpentyloxycarbonyl, 1-Propylbutyloxycarbonyl und Octyloxycarbonyl, insbesondere $C_1$-$C_4$-Alkoxycarbonyl wie Methoxycarbonyl, Ethoxycarbonyl, Propoxycarbonyl, 1-Methylethoxycarbonyl und 1-Methylpropoxycarbonyl;

Phenyl, Phenoxy, Phenylcarbonyl, 2-, 3-, 4-Fluorphenyl, 2-, 3-, 4-Chlorphenyl, 2-, 3-, 4-Methylphenyl, 2-, 3-, 4-Methylphenoxy, 2-, 3-, 4-Methylphenylcarbonyl, 2-, 3-, 4-Trifluormethylphenyl, 2-, 3-, 4-Trifluormethylphenoxy, 2-, 3-, 4-Trifluormethylphenylcarbonyl, 2-, 3-, 4-Methoxyphenyl, 2-, 3-, 4-Methoxyphenoxy, 2-, 3-, 4-Methylthiophenyl;

$C_2$-$C_6$-Alkyl, insbesondere $C_2$-$C_4$-Alkyl, das in 2-Stellung substituiert ist durch $C_1$-$C_6$-Alkoxyimino, insbesondere $C_1$-$C_4$-Alkoxyimino wie Methoxy-, Ethoxy-, Propoxy- und Butoxyimino; $C_3$-$C_6$-Alkenyloxyimino, vorzugsweise $C_3$-$C_4$-Alkenyloxyimino wie 2-Propenyloxyimino, 2-Butenyloxyimino, 3-Butenyloxyimino; $C_3$-$C_6$-Halogenalkenyloxyimino, insbesondere $C_3$-Halogenalkenyloxyimino wie 3,3-Dichlor-2-propenyloxyimino und 2,3,3-Trichlor-2-propenyloxyimino oder Benzyloxyimino;

$C_3$-$C_6$-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere $C_3$-$C_4$-Alkenyl wie 2-Propenyl, 2-Butenyl, 1-Methyl-2-propenyl, 2-Methyl-2-propenyl, sowie 3-Methyl-2-butenyl und 3-Methyl-2-pentenyl;

$C_3$-$C_6$-Alkinyl wie 2-Propinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, insbesondere $C_3$-$C_4$-Alkinyl wie 2-Propinyl, 2-Butinyl und 3-Butinyl, wobei 2-Propinyl ein bis drei, die Alkenyl- und restlichen Alkinylgruppen ein bis fünf Halogenatome, insbesondere Fluor und Chlor tragen können;

Phenyl, ein- bis dreifach durch $C_1$-$C_4$-Alkyl wie Methyl, Ethyl, Propyl, Butyl oder $C_1$-$C_4$-Alkoxy wie Methoxy, Ethoxy, Propoxy, Butoxy oder ein- bis fünffach durch Halogen, insbesondere Fluor und Chlor, substituiertes Phenyl;

$-N=CR^6R^7$, wobei $R^6$ und $R^7$ folgende Bedeutung haben:

$R^6$, $R^7$ $C_1$-$C_{20}$-Alkyl, vorzugsweise $C_1$-$C_{15}$-Alkyl, insbesondere $C_1$-$C_{10}$-Alkyl wie oben genannt, welches einen Phenylrest, $C_1$-$C_4$-Alkoxy wie oben genannt und/oder $C_1$-$C_4$-Alkylthio wie oben genannt tragen kann;

Phenyl, oder gemeinsam $C_3$-$C_{12}$-Alkylen, vorzugsweise $C_4$-$C_7$-Alkylen, welches ein bis drei $C_1$-$C_3$-Alkylgruppen, vorzugsweise Methyl- oder Ethylgruppen tragen kann;

einen Rest $-OR^8$, in dem $R^8$ folgende Bedeutung hat:

$R^8$ Wasserstoff, das Kation eines Alkalimetalls wie Natrium, Kalium, das Kation eines Erdalkalimetalls wie Magnesium oder Calcium, Ammonium oder ein organisches Ammoniumion wie Tri-($C_1$-$C_4$)-alkylammonium, bevorzugt Triethylammonium und Tributylammonium.

$R^2$, $R^3$ $C_1$-$C_4$-Alkyl wie Methyl, Ethyl, Propyl, 1-Methylethyl, Butyl, 1-Methylpropyl, 2-Methylpropyl und 1,1-Dimethylethyl;

$C_1$-$C_2$-Halogenalkyl wie Halogenalkyl wie Chlormethyl, Dichlormethyl, Trichlormethyl, Chlorfluormethyl, Fluormethyl, Difluormethyl, Trifluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;

$C_1$-$C_4$-Alkoxy wie Methoxy, Ethoxy, Propyloxy, 1-Methylethoxy, Butyloxy, 1-Methyl-propoxy, 2-Methylpropyloxy und 1,1-Dimethylethoxy;

$C_1$-$C_2$-Halogenalkoxy wie Chlormethyloxy, Dichlcrmethyloxy, Trichlormethyloxy, Fluormethoxy, Difluormethoxy, Trifluormethoxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyl, insbesondere Trifluormethoxy;

$C_1$-$C_4$-Alkylamino wie Methylamino, Ethylamino, Propylamino, 1-Methylethylamino, Butylamino, 1-Methylpropylamino, 2-Methylpropylamino, 1,1-Dimethylethylamino;

Di-($C_1$-$C_4$-alkyl)amino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)-amino, N-Ethyl-N-(2-methylpropyl)amino,N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino,N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino, insbesondere Di($C_1$-$C_2$-alkyl)amino wie N,N-Dimethylamino, N,N-Diethylamino und N-Methyl-N-ethylamino;

$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio,

$R^4$ $C_1$-$C_8$-Alkyl wie Methyl, Ethyl, Propyl, 1-Methylethyl, Butyl, 1-Methylpropyl, 2-Methylpropyl, 1,1-Dimethylkethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl, Octyl, insbesondere $C_1$-$C_4$-Alkyl wie Methyl, Ethyl, Propyl, 1-Methylethyl, Butyl, 1-Methylpropyl, 2-Methylpropyl und 1,1-Dimethylethyl,

$C_3$-$C_6$-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methl-2-butenyl, 1-Methyl-4-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere $C_3$-$C_4$-Alkenyl wie 2-Propenyl, 2-Butenyl, 3-Butenyl, 1-Methylpropenyl, 2-Methyl-2-propenyl;

$C_3$-$C_6$-Halogenalkenyl wie 2-Chlorallyl, E-, Z-3-Chlorallyl;

Benzyl, ein- bis dreifach im Phenylring durch $C_1$-$C_3$-Alkyl, Halogen oder $C_1$-$C_4$-Alkoxy substituiertes Benzyl wie 2-, 3-, 4-Methylbenzyl, 2-, 3-, 4-Fluorbenzyl, 2-, 3-, 4-Chlorbenzyl, 2-,

3-, 4-Methoxybenzyl;

X Sauerstoff, Schwefel;

Z Stickstoff, die Methingruppe =CH-.

Die Ausgangsverbindungen II können aufgrund der C=N-Doppelbindung als E/Z-Isomerengemische oder aber als reine E- oder Z-Isomere vorliegen, so daß die entsprechenden Verbindungen I entweder als E/Z-Isomerengemische oder als E- oder Z-Isomere anfallen. Sowohl die einzelnen isomeren Verbindungen als auch ihre Gemische werden von der Erfindung umfaßt.

Man erhält die Verbindungen I beispielsweise, indem man ein aromatisches Carbonsäurederivat II mit einer Verbindung III in Gegenwart einer Base in an sich bekannter Weise umsetzt.

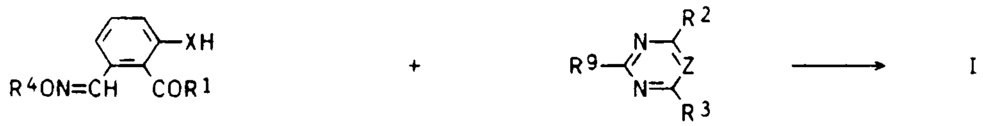

II III

$R^9$ bedeutet eine nucleofuge Abgangsgruppe, beispielsweise Halogen wie Chlor, Brom oder Iod, Aryl- oder Alkylsulfonyl wie Toluolsulfonyl oder Methylsulfonyl oder eine andere äquivalente Abgangsgruppe.

Die Verbindungen II erhält man im allgemeinen nach bekannten Methoden wie Ether- oder Esterspaltung (falls $R^{11} \neq H$) aus den Verbindungen IV, welche aus den entsprechenden bekannten Aldehyden zugänglich sind:

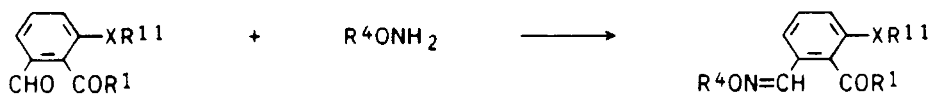

IV

Verbindungen der Formel III mit einem reaktionsfähigen Substituenten $R^9$ sind bekannt oder nach bekannten Methoden leicht zu erhalten.

Als Basen können Alkali- oder Erdalkalimetallhydride wie NaH oder $CaH_2$, Alkalimetallhydroxide wie NaOH oder KOH, Alkalimetallalkoholate wie Kalium-tert.-butylat, Alkalimetallcarbonate wie $Na_2CO_3$ oder $K_2CO_3$, Alkalimetallamide wie $NaNH_2$ oder Lithiumdiisopropylamid oder tertiäre Amine wie Triethylamin Verwendung finden. Bei Einsatz einer anorganischen Base kann man einen Phasentransferkatalysator wie ein organisches Ammoniumsalz oder einen Kronenether zusetzen, wodurch die Reaktion häufig beschleunigt wird.

Soweit es sich bei den Verbindungen 1 um die Carbonsäuren I' handelt ($R^8$ = H), können hieraus die anderen definitionsgemäßen Verbindungen z.B. auch dadurch hergestellt werden, daß man die Carbonsäure I' zunächst auf übliche Weise in eine aktivierte Form wie ein Halogenid, bevorzugt das Säurechlorid, oder Imidazolid überführt und diese dann mit einem Alkohol $R^5OH$ wie Ethanol, Propargylalkohol oder Allylalkohol, einem Di- oder Tri-Azol wie Imidazol oder 1,2,4-Triazol oder N-Hydroxysuccinimid umsetzt. Diese beiden Schritte lassen sich beispielsweise auch dadurch vereinfachen, daß man die Carbonsäure in Gegenwart eines wasserabspaltenden Mittels wie eines Carbodiimids oder eines Phosphonsäureanhydrids auf die Hydroxylverbindung einwirken läßt.

Weiterhin kann man die Carbonsäuren I' zunächst in an sich bekannter Weise in ein Salz, bevorzugt ein Alkalimetallsalz, überführen und dieses dann mit einer Verbindung $R^{10}$-$R^{5'}$ zu den Verbindungen I umsetzen. Bei dieser Reaktion können die in der Umsetzung der Verbindungen II und III verwendeten Basen ebenfalls eingesetzt werden. In der verwendeten Verbindung $R^{10}$-$R^{5'}$ steht $R^{10}$ für eine nucleofuge Abgangsgruppe wie Chlor, Brom, Iod, Aryl- oder Alkylsulfonyl wie Toluolsulfonyl oder Methylsulfonyl und $R^{5'}$ für die unter $R^5$ genannten Reste außer unsubstituiertes und substituiertes Phenyl und die Gruppe $-N=CR^6R^7$.

Die verwendeten Verbindungen $R^{10}$-$R^{5'}$ sind bekannt oder lassen sich nach bekannten Methoden herstellen.

Die erfindungsgemäßen herbiziden und wachstumsregulierenden Verbindungen I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon und Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.% Wirkstoff. Die Wirkstoffe werden dabei in einer Reinheit von 90 % bis 100 %, vorzugsweise 95 % bis 100 % (nach NMR-Spektrum) eingesetzt.

Beispiele für solche Zubereitungen sind:

I. eine Lösung aus 90 Gew.-Teilen der Verbindung Nr. 1 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. 2, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. 9, 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 10, 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 20 Gew.-Teilen der Verbindung Nr. 17, 3 Gew.-Teilen des Natriumsalzes der Diisobutylnaphtalin-α-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in 20.000 Gew.-Teilen Wasser erhält man eine Spritzbrühe, die 0,1 Gew.-% des Wirkstoffs enthält.

VI. eine innige Mischung aus 3 Gew.-Teilen der Verbindung Nr. 23 und 97 Gew.-Teilen feinteiligem

Kaolin; dieses Stäubemittel enthält 3 Gew.-% Wirkstoff;

VII. eine innige Mischung aus 30 Gew.-Teilen der Verbindung Nr. 1, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile ölige Dispersion aus 20 Gew.-Teilen der Verbindung Nr. 2, 2 Gew.-Teilen des Calciumsalzes der Dodecylbenzolsulfonsäure, 8 Gew.-Teilen Fettalkohol-polyglykolether, 2 Gew.-Teilen des Natriumsalzes eines Phenolsulfonsäure-harnstoff-formaldehydKondensates und 68 Gew.-Teilen eines paraffinischen Mineralöls.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an herbizidem Wirkstoff betragen je nach Bekämpfungsziel, Jahreszeit, Zielpflanzen und Wachstumsstadium 0,001 bis 3,0, vorzugsweise 0,005 bis 2,0 kg/ha aktive Substanz (a.S.).

Die wachstumsregulierend wirkenden Carbonsäurederivate der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren z.B. (Samenbeize, Bodenbehandlung, Blattapplikation oder Stamminjektion bei Bäumen)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die

generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren. Dabei können die Benzaldoximetherderivate I Ertragssteigerungen durch Eingriffe in den pflanzlichen Stoffwechsel bzw. durch Förderung oder Hemmung des vegetativen und/oder des generativen Wachstums bewirken.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird und
- das Mikroklima im Pflanzenbestand durch einen kompakteren Wuchs günstig beeinflußt wird.

Die erfindungsgemäß zu verwendenden Wirkstoffe der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g, vorzugsweise 0,01 bis 10 g, je kilogramm Saatgut, benötigt.

Für die Blatt- und Bodenbehandlung sind im allgemeinen Gaben von 0,001 bis 10 kg/ha, bevorzugt 0,01 bis 3 kg/ha, insbesondere 0,01 bis 0,5 kg/ha als ausreichend zu betrachten.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |

| Botanischer Name | Deutscher Name |
|---|---|
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohne |
| Picea abies | Rotfichte |
| Pinus spp. | Kiefer |
| Pisum sativum | Gertenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die erfindungsgemäßen Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazin, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffderivate, (Hetero)-aryloxy-phenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von

Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I verwendet. Die so erhaltenen Verbindungen sind in der nachstehenden Tabelle mit physikalischen Angaben aufgeführt. Verbindungen ohne diese Angaben lassen sich analog zu den entsprechenden Ausgangsverbindungen synthetisieren. Die in der Tabelle wiedergegebenen Strukturen beschreiben besonders bevorzugte Wirkstoffe der Formel I.

A) Synthese der Verbindungen IV

Beispiel 1

2-Ethoxyiminomethyl-6-hydroxybenzoesäureethylester

a) 2-Acetoxy-6-formylbenzoesäureethylester

38,1 g N-Methylmorpholin-N-oxid wurden in 1,5 l Chloroform vorgelegt und azeotrop getrocknet. Dann wurde granuliertes Molekularsieb 4A (1/8-inch-Kügelchen) (250 ml volumetrisch abgemessen) zugegeben und über Nacht bei Raumtemperatur gerührt. Ein Drittel der Lösung wurde zur späteren Verwendung separat abgefüllt. Dann wurden 47,2 g 2-Acetoxy-6-brommethylbenzoesäureethylester (s. T. Eicher et al., Synthesis 1988, S. 525-529) zugegeben. Man rührte 3,5 h bei 55-60 °C, gab das zurückgestellte Drittel Oxidationsmittel zu, rührte weitere 3 h bei derselben Temperatur und fügte dann nochmals Oxidationsmittel zu (21,2 g N-Methylmorpholin-N-oxid in 1,1 l Chloroform gelöst und wie oben beschrieben getrocknet, wobei eine äquivalente Menge Molekularsieb verwendet wurde). Nach 3 h bei 55-60 °C wurde vom Molekularsieb dekantiert und letzteres 4 mal mit je 400 ml Methylenchlorid gewaschen. Die vereinigten organischen Phasen wurden viermal mit je 150 ml 5 %iger Phosphorsäure und dann dreimal mit derselben Menge gesättigter Kochsalzlösung gewaschen, über Natriumsulfat getrocknet und im Vakuum eingeengt. Ausbeute: 27 g

b) 2-Acetoxy-6-ethoxyiminomethylbenzoesäureethylester

10,0 g des unter a) erhaltenen 2-Acetoxy-6-formylbenzoesäureethylesters wurden in 320 ml Toluol vorgelegt und mit Molekularsieb 4A (190 ml volumetrisch abgemessen) versetzt. Dann wurden 2,8 g Ethoxyamin zugegeben. Man rührte 30 min bei Raumtemperatur, 30 min bei 45 °C und dann über Nacht bei Raumtemperatur. Dann wurde vom Molekularsieb dekantiert und letzteres sorgfältig mit Toluol nachgewaschen. Nach dem Einengen im Vakuum verblieben 8,8 g Produkt als Öl.

c) 2-Ethoxyiminomethyl-6-hydroxybenzoesäureethylester

5,0 g 2-Acetoxy-6-ethoxyiminomethylbenzoesäureethylester wurden in 100 ml Methanol gelöst und mit einer Lösung von 1,5 g Natriumhydrogencarbonat in 120 ml Wasser versetzt. Man rührte 2,5 h bei 45 °C und goß die Reaktionsmischung dann zu 600 ml 5 %ige Orthophosphorsäure. Anschließend wurde mit Essigsäureethylester extrahiert, über Natriumsulfat getrocknet und im Vakuum eingeengt. Ausbeute: 3,6 g.

$^1$H-HMR ($CDCl_3$): $\delta$ = 1,33 (t); 1,42 (t); 4,22 (q); 4,42 (q), 7,02 (d); 7,23 (d); 7,41 (t); 8,61 (s); 10,20 (s).

Analog dazu wurde hergestellt:

Beispiel 2

2-t.-Butoxyiminomethyl-6-hydroxybenzoesäureethylester

$^1$H-NMR ($CDCl_3$): $\delta$ = 1,36 (s); 1,42 (t); 4,42 (q); 7,00-7,43 (m), 8,60 (s); 11,15 (s).

Beispiel 3

2-Ethoxyiminomethyl-6-hydroxybenzoesäure

8,6 g 2-Acetoxy-6-ethoxyiminomethylbenzoesäureethylester (s. Beispiel 1) wurden in 60 ml Methanol

und 20 ml Wasser gelöst und mit 4,9 g 50 %iger Natronlauge versetzt. Es wurde 5 h bei 75 °C gerührt, das Methanol weitgehend im Vakuum entfernt und mit 200 ml kaltem Wasser verdünnt. Dann wurde das Produkt mit 10 %iger Salzsäure gefällt, abgetrennt, mit wenig Wasser gewaschen und schließlich mit n-Pentan dreimal aufgerührt und abfiltriert. Anschließend wurde im Vakuum getrocknet. Ausbeute 3,7 g.
$^1$H-NMR ($CDCl_3$): δ = 1,38 (t); 4,30 (q); 7,05-7,50 (m), 8,65 (s).
Entsprechend wurden hergestellt:

Beispiel 4

2-Allyloxyiminomethyl-6-hydroxybenzoesäure

$^1$H-NMR ($CDCl_3$): δ = 4,70 (d); 5,25-5,45 (m); 5,95-6,15 (m); 7,05-7,55 (m), 8,65 (s).

Beispiel 5

2-t.Butoxyiminomethyl-6-hydroxybenzoesäure

$^1$H-NMR ($CDCl_3$): δ = 1,40 (s); 7,10-7,55 (m); 8,47 (s).

Beispiel 6

2-(4-Chlorbenzyloxy)iminomethyl-6-hydroxybenzoesäure

$^1$H-HMR ($CDCl_3$): δ = 5,18 (s); 7,05-7,60 (m), 8,67 (s).

B) Synthese der Verbindungen I

Beispiel 7

2-t.-Butoxyiminomethyl-6-(4,6-dimethoxypyrimidin-2-yloxy)benzoesäureethylester (s. Tabelle, Nr. 9)

2,00 g 2-t.-Butoxyiminomethyl-6-hydroxybenzoesäureethylester wurden in 50 ml Dimethylsulfoxid gelöst, mit 0,90 g Kalium-tert.-butylat versetzt und 10 min bei Raumtemperatur nachgerührt. Dann wurden 1,75 g 4,6-Dimethoxy-2-methylsulfonylpyrimidin zugegeben und 2 h bei Raumtemperatur nachgerührt. Die Reaktionsmischung wurde zu 600 ml 5 %iger Orthophosphorsäure gegeben, mit Essigsäureethylester extrahiert und die organische Phase mit Wasser gewaschen. Nach dem Trocknen über Natriumsulfat wurde im Vakuum eingeengt. Zur weiteren Reinigung wurde an Kieselgel mit Toluol/Hexan als Elutionsmittel chromatographiert. Ausbeute: 2,4 g.
$^1$H-NMR ($CDCl_3$): δ = 1,15 (t); 1,33 (s); 3,80 (s); 4,22 (q); 5,78 (s); 7,25-7,65 (m), 8,23 (s).

Beispiel 8

2-t.-Butoxyiminomethyl-6-(4,6-dimethoxypyrimidin-2-yloxy)benzoesäure (s. Tabelle, Nr. 10)

2,65 g 2-t.-Butoxyiminomethyl-6-hydroxybenzoesäure wurden in 60 ml Dimethylsulfoxid gelöst, mit 2,63 g Kalium-tert.-butylat versetzt und 20 min bei Raumtemperatur nachgerührt. Dann wurden 2,56 g 4,6-Dimethoxy-2-methylsulfonylpyrimidin zugegeben und 30 min bei Raumtemperatur nachgerührt. Die Reaktionsmischung wurde zu 800 ml 5 %ige Orthophosphorsäure gegeben, der Niederschlag abfiltriert, mit Wasser gewaschen und im Vakuum getrocknet. Zur Reinigung wurde in Diethylether gelöst, dann filtriert, das Filtrat im Vakuum eingeengt und der Rückstand mit Cyclohexan gewaschen. Nach dem Entfernen des restlichen Lösungsmittels im Vakuum verblieben 1,9 g Feststoff vom Schmp. 63 °C.

Tabelle: Benzaldoximetherderivate I (mit $R^3 = OCH_3$)

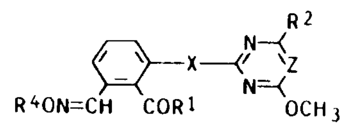

| Verb. Nr. | $R^1$ | $R^2$ | $R^4$ | X | Z | phys. Daten* |
|---|---|---|---|---|---|---|
| 01) | OH | $OCH_3$ | Ethyl | O | CH | Fp. = 121°C |
| 02) | Ethoxy | $OCH_3$ | Ethyl | O | CH | $n_D^{23}$ = 1,5470 |
| 03) | 2-Propaniminoxy | $OCH_3$ | Ethyl | O | CH | |
| 04) | Methylthiomethyloxy | $OCH_3$ | Ethyl | O | CH | |
| 05) | Propargyloxy | $OCH_3$ | Ethyl | O | CH | |
| 06) | OH | $OCH_3$ | Ethyl | O | N | |
| 07) | OH | $OCH_3$ | Ethyl | S | CH | |
| 08) | OH | $CF_3$ | Ethyl | O | CH | |
| 09) | Ethoxy | $OCH_3$ | t.-Butyl | O | CH | δ = 1,15 (t); 1,33 (s); 3,80 (s); 4,22 (q); 5,78 (s); 7,25-7,65 (m); 8,23 (s) |
| 10) | OH | $OCH_3$ | t.-Butyl | O | CH | Fp. = 63°C |
| 11) | OH | $OCH_3$ | Methyl | O | CH | |
| 12) | Allyloxy | $OCH_3$ | Methyl | O | CH | |
| 13) | OH | $OCH_3$ | n-Propyl | O | CH | |
| 14) | OH | $OCH_3$ | iso-Propyl | O | CH | |

Tabelle (Fortsetzung)

| Verb. Nr. | $R^1$ | $R^2$ | $R^4$ | X | Z | phys. Daten* |
|---|---|---|---|---|---|---|
| 15) | OH | $OCH_3$ | iso-Butyl | O | CH | |
| 16) | OH | $OCH_3$ | sek.-Butyl | O | CH | |
| 17) | OH | $OCH_3$ | Allyl | O | CH | Fp. = 148°C |
| 18) | Propargyloxy | $OCH_3$ | Allyl | O | CH | |
| 19) | OH | $OCH_3$ | E-3-Chlorallyl | O | CH | |
| 20) | OH | $OCH_3$ | Z-3-Chlorallyl | O | CH | |
| 21) | OH | $OCH_3$ | 2-Chlorallyl | O | CH | |
| 22) | OH | $OCH_3$ | Benzyl | O | CH | |
| 23) | OH | $OCH_3$ | p-Chlorbenzyl | O | CH | Fp. = 144°C |
| 24) | OH | $OCH_3$ | o-Methylbenzyl | O | CH | |
| 25) | 2-Propaniminoxy | $OCH_3$ | Methyl | O | CH | |
| 26) | Benzyloxy | $OCH_3$ | Methyl | O | CH | |
| 27) | Ethoxycarbonylmethyloxy | $OCH_3$ | Ethyl | O | CH | |
| 28) | 1-Imidazolyloxy | $OCH_3$ | Ethyl | O | CH | |
| 29) | 2-(Methoxyimino)-ethyloxy | $OCH_3$ | Ethyl | O | CH | |
| 30) | 2-(Ethoxyimino)-propyloxy | $OCH_3$ | Ethyl | O | CH | |
| 31) | 2-(3-Allyloxyimino)-ethyloxy | $OCH_3$ | Ethyl | O | CH | |

* Fp.: Schmelzpunkt, $n_D$: Brechungsindex, δ: $^1H$-NMR - chemische Verschiebung in ppm (ausgewählte Signale).

Anwendungsbeispiele zur herbiziden Wirkung

Die herbizide Wirkung einer Verbindung I ließ sich durch Gewächshausversuche zeigen:

Zur Aufzucht der Testpflanzen dienten Plastikblumentöpfe mit 300 $cm^3$ Inhalt und lehmigem Sand mit etwa

3 % Humus als Substrat. Die Samen der Testpflanzen wurden nach Arten getrennt eingesät.

Zum Zweck der Nachauflaufbehandlung wurden die Testpflanzen je nach Wuchsform erst bis zu einer Wuchshöhe von 3 bis 15 cm angezüchtet und erst dann mit den in Wasser suspendierten oder emulgierten Wirkstoffen behandelt. Die Testpflanzen wurden dafür entweder direkt gesät und in den gleichen Gefäßen aufgezogen oder sie wurden als Keimpflanzen getrennt gezüchtet und einige Tage vor der Behandlung in die Versuchsgefäße verpflanzt. Die Aufwandmenge für die Nachauflaufbehandlung betrug 0,25 kg Wirkstoff/ha.

Die Versuchsgefäße wurden im Gewächshaus aufgestellt, wobei für wärmeliebende Arten wärmere Bereiche (20 bis 35 °C) und für solche gemäßigter Klimate 10 bis 25 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürzung | Lateinischer Name | Deutscher Name | Englischer Name |
|---|---|---|---|
| AMARE | Amaranthus retroflexus | zurückgekrümmter Fuchsschwanz | redroot pigweed |
| CHEAL | Chenopodium album | Weißer Gänsefuß | lambsquarters (goose-foot) |
| SOLNI | Solanum nigrum | Schwarzer Nachtschatten | black nightshade |

Mit 0,25 kg/ha a.S. im Nachauflaufverfahren eingesetzt, lassen sich mit der Beispielsverbindung 1 (s. Tabelle) breitblättrige unerwünschte Pflanzen sehr gut bekämpfen. Bei den Testpflanzen AMARE, CHEAL und SOLNI traten jeweils 100 %ige Schädigungen auf.

Die Beispielsverbindung 2 (s. Tabelle) wurde an folgenden Arten im Gewächshaus getestet:

| Abkürzung | Lateinischer Name | Deutscher Name |
|---|---|---|
| TRZAS | Triticum aestivum | Sommerweizen |
| GALAP | Galium aparine | Kettenlabkraut |
| POLPE | Polygonum persicaria | Flohknöterich |
| EPHHL | Emphorbia heterophylla | Wolfsmilchart |

und zeigte im Nachauflaufverfahren bei einer Aufwandmenge von 0,125 kg/ha a.S. eine sehr gute Bekämpfung der unerwünschten Testpflanzen während die Beispielkultur Weizen kaum Schädigungen aufwies.

## Patentansprüche

**1.** Benzaldoximetherderivate der Formel I,

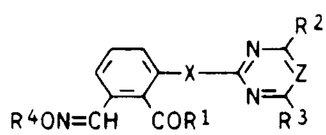

I

in der die Substituenten folgende Bedeutung haben:

$R^1$ Succinimidoxy;

einen 5-gliedrigen Heteroaromaten, enthaltend zwei oder drei Stickstoffatome, welcher ein bis zwei Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

einen Rest $-OR^5$, in dem

$R^5$ eine $C_3$-$C_{12}$-Cycloalkylgruppe, welche ein bis drei $C_1$-$C_4$-Alkylreste tragen kann;

eine $C_1$-$C_{10}$-Alkylgruppe, welche ein bis fünf Halogenatome und/oder einen der folgenden Reste tragen kann:

$C_1$-$C_4$-Alkoxy, $C_1$-$C_4$-Alkylthio, Cyano, $C_1$-$C_8$-Alkylcarbonyl, $C_3$-$C_{12}$-Cycloalkyl, $C_1$-$C_8$-Alkoxycarbonyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

eine $C_2$-$C_6$-Alkylgruppe, welche in der 2-Position einen der folgenden Reste trägt: $C_1$-$C_6$-Alkoxyimino, $C_3$-$C_6$-Alkenyloxyimino, $C_3$-$C_6$-Halogenalkenyloxyimino oder Benzyloxyimino;

eine $C_3$-$C_6$-Alkenyl- oder eine $C_3$-$C_6$-Alkinylgruppe, wobei diese Gruppen ihrerseits ein bis fünf Halogenatome tragen können;

unsubstituiertes oder ein- bis dreifach durch $C_1$-$C_4$-Alkyl oder $C_1$-$C_4$-Alkoxy substituiertes oder ein- bis fünffach durch Halogen substituiertes Phenyl; oder

den Rest $-N=CR^6R^7$, in dem

$R^6$ und $R^7$ $C_1$-$C_{20}$-Alkyl, welches einen Phenylrest, eine $C_1$-$C_4$-Alkoxy- und/oder eine $C_1$-$C_4$-Alkylthiogruppe tragen kann, Phenyl oder gemeinsam eine $C_3$-$C_{12}$-Alkylenkette, welche ein bis drei $C_1$-$C_3$-Alkylgruppen tragen kann, bedeuten;

bedeutet, oder

einen Rest $-OR^8$, in dem

$R^8$ für Wasserstoff, ein Alkalimetallkation, das Äquivalent eines Erdalkalimetallkations, Ammonium oder ein organisches Ammoniumion steht;

$R^2$, $R^3$ $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylamino, Di-($C_1$-$C_4$-alkyl)-amino und/oder $C_1$-$C_4$-Alkylthio;

$R^4$ $C_1$-$C_8$-Alkyl, $C_3$-$C_6$-Alkenyl, $C_3$-$C_6$-Halogenalkenyl oder Benzyl, welches im Phenylring ein bis drei der folgenden Substituenten tragen kann: $C_1$-$C_3$-Alkyl, Halogen, $C_1$-$C_4$-Alkoxy;

X Sauerstoff oder Schwefel;

Z Stickstoff oder eine Methingruppe $=CH-$.

2. Verfahren zur Herstellung der Verbindungen der Formel I gemäß Anspruch 1, dadurch gekennzeichnet, daß man eine Verbindung der Formel II

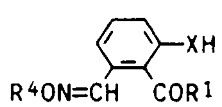

II

in an sich bekannter Weise mit einem Heterocyclus der Formel III

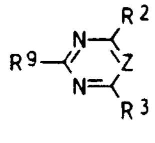

III

in der $R^9$ für eine nucleofuge Abgangsgruppe steht, in Gegenwart einer Base umsetzt.

3. Verfahren zur Herstellung der Verbindungen der Formel I gemäß Anspruch 1, dadurch gekennzeichnet, daß man die freie Carbonsäure der Formel I'

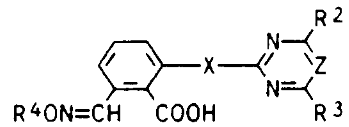

I'

zunächst in an sich bekannter Weise in das Halogenid oder eine andere aktivierte Form der Carbonsäure überführt und diese mit einem Alkohol $R^5OH$, einem Di- oder Triazol oder N-Hydroxysuccinimid umsetzt.

**4.** Verfahren zur Herstellung der Verbindungen der Formel I gemäß Anspruch 1, dadurch gekennzeichnet, daß man die freie Carbonsäure I' zunächst in an sich bekannter Weise in ein Salz überführt und dieses dann mit einer Verbindung $R^{10}$ - $R^{5'}$ umsetzt, wobei $R^{10}$ für eine nucleofuge Abgangsgruppe steht und $R^{5'}$ folgende Bedeutung hat:

eine $C_3$-$C_{12}$-Cycloalkylgruppe, welche ein bis drei $C_1$-$C_4$-Alkylreste tragen kann;

eine $C_1$-$C_{10}$-Alkylgruppe, welche ein bis fünf Halogenatome und/oder einen der folgenden Reste tragen kann:

$C_1$-$C_4$-Alkoxy, $C_1$-$C_4$-Alkylthio, Cyano, $C_1$-$C_8$-Alkylcarbonyl, $C_3$-$C_{12}$-Cycloalkyl, $C_1$-$C_8$-Alkoxycarbonyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

eine $C_2$-$C_6$-Alkylgruppe, welche in der 2-Position einen der folgenden Reste trägt: $C_1$-$C_6$-Alkoxyimino, $C_3$-$C_6$-Alkenyloxyimino, $C_3$-$C_6$-Halogenalkenyloxyimino oder Benzyloxyimino;

eine $C_3$-$C_6$-Alkenyl- oder eine $C_3$-$C_6$-Alkinylgruppe, wobei diese Gruppen ihrerseits ein bis fünf Halogenatome tragen können.

**5.** Verbindungen der Formel IV,

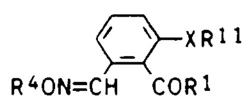

IV

in der $R^{11}$ für Wasserstoff, Acetyl oder Methyl steht.

**6.** Herbizides Mittel, enthaltend eine Verbindung der Formel I gemäß Anspruch 1.

**7.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Derivates I gemäß Anspruch 1 behandelt.

**8.** Verwendung von Benzaldoximetherderivaten der Formel I gemäß Anspruch 1 als Herbizide.

**9.** Mittel zur Beeinflussung des Pflanzenwuchses, enthaltend ein Benzaldoximetherderivat der Formel I gemäß Anspruch 1.

**10.** Verfahren zur Regulierung des Pflanzenwuchses, dadurch gekennzeichnet, daß man eine regulativ wirksame Menge eines Benzaldoximetherderivates der Formel I gemäß Anspruch 1 auf die Samen, die Pflanzen und/oder deren Lebensraum einwirken läßt.

**Patentansprüche für folgenden Vertragsstaat : ES**

**1.** Herbizides Mittel, enthaltend neben üblichen inerten Zusatzstoffen Benzaldoximetherderivate der Formel I,

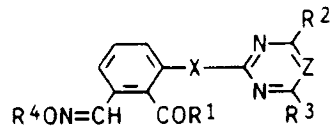

I

in der die Substituenten folgende Bedeutung haben:

$R^1$ Succinimidoxy;

einen 5-gliedrigen Heteroaromaten, enthaltend zwei oder drei Stickstoffatome, welcher ein bis zwei Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

einen Rest -$OR^5$, in dem

$R^5$ eine $C_3$-$C_{12}$-Cycloalkylgruppe, welche ein bis drei $C_1$-$C_4$-Alkylreste tragen kann;

eine $C_1$-$C_{10}$-Alkylgruppe, welche ein bis fünf Halogenatome und/oder einen der folgenden Reste tragen kann:

$C_1$-$C_4$-Alkoxy, $C_1$-$C_4$-Alkylthio, Cyano, $C_1$-$C_8$-Alkylcarbonyl, $C_3$-$C_{12}$-Cycloalkyl, $C_1$-$C_8$-Alkoxycarbonyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

eine $C_2$-$C_6$-Alkylgruppe, welche in der 2-Position einen der folgenden Reste trägt: $C_1$-$C_6$-Alkoxyimino, $C_3$-$C_6$-Alkenyloxyimino, $C_3$-$C_6$-Halogenalkenyloxyimino oder Benzyloxyimino;

eine $C_3$-$C_6$-Alkenyl- oder eine $C_3$-$C_6$-Alkinylgruppe, wobei diese Gruppen ihrerseits ein bis fünf Halogenatome tragen können;

unsubstituiertes oder ein- bis dreifach durch $C_1$-$C_4$-Alkyl oder $C_1$-$C_4$-Alkoxy substituiertes oder ein- bis fünffach durch Halogen substituiertes Phenyl; oder

den Rest $-N=CR^6R^7$, in dem

$R^6$ und $R^7$ $C_1$-$C_{20}$-Alkyl, welches einen Phenylrest, eine $C_1$-$C_4$-Alkoxy- und/oder eine $C_1$-$C_4$-Alkylthiogruppe tragen kann, Phenyl oder gemeinsam eine $C_3$-$C_{12}$-Alkylenkette, welche ein bis drei $C_1$-$C_3$-Alkylgruppen tragen kann, bedeuten;

bedeutet, oder

einen Rest -$OR^8$, in dem

$R^8$ für Wasserstoff, ein Alkalimetallkation, das Äquivalent eines Erdalkalimetallkations, Ammonium oder ein organisches Ammoniumion steht;

$R^2$, $R^3$ $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylamino, Di-($C_1$-$C_4$-alkyl)-amino und/oder $C_1$-$C_4$-Alkylthio;

$R^4$ $C_1$-$C_8$-Alkyl, $C_3$-$C_6$-Alkenyl, $C_3$-$C_6$-Halogenalkenyl oder Benzyl, welches im Phenylring ein bis drei der folgenden Substituenten tragen kann: $C_1$-$C_3$-Alkyl, Halogen, $C_1$-$C_4$-Alkoxy;

X Sauerstoff oder Schwefel;

Z Stickstoff oder eine Methingruppe =CH-.

**2.** Verfahren zur Herstellung der Verbindungen der Formel I gemäß Anspruch 1, dadurch gekennzeichnet, daß man eine Verbindung der Formel II

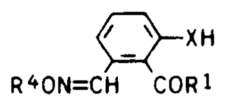

II

in an sich bekannter Weise mit einem Heterocyclus der Formel III

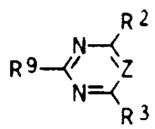

III

in der $R^9$ für eine nucleofuge Abgangsgruppe steht, in Gegenwart einer Base umsetzt.

3. Verfahren zur Herstellung der Verbindungen der Formel I gemäß Anspruch 1, dadurch gekennzeichnet, daß man die freie Carbonsäure der Formel I'

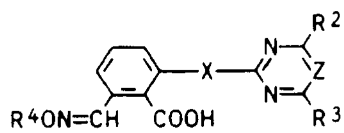

I'

zunächst in an sich bekannter Weise in das Halogenid oder eine andere aktivierte Form der Carbonsäure überführt und diese mit einem Alkohol $R^5OH$, einem Di- oder Triazol oder N-Hydroxysuccinimid umsetzt.

4. Verfahren zur Herstellung der Verbindungen der Formel I gemäß Anspruch 1, dadurch gekennzeichnet, daß man die freie Carbonsäure I' zunächst in an sich bekannter Weise in ein Salz überführt und dieses dann mit einer Verbindung $R^{10}$ - $R^{5'}$ umsetzt, wobei $R^{10}$ für eine nucleofuge Abgangsgruppe steht und $R^{5'}$ folgende Bedeutung hat:

eine $C_3$-$C_{12}$-Cycloalkylgruppe, welche ein bis drei $C_1$-$C_4$-Alkylreste tragen kann;

eine $C_1$-$C_{10}$-Alkylgruppe, welche ein bis fünf Halogenatome und/oder einen der folgenden Reste tragen kann:

$C_1$-$C_4$-Alkoxy, $C_1$-$C_4$-Alkylthio, Cyano, $C_1$-$C_8$-Alkylcarbonyl, $C_3$-$C_{12}$-Cycloalkyl, $C_1$-$C_8$-Alkoxycarbonyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

eine $C_2$-$C_6$-Alkylgruppe, welche in der 2-Position einen der folgenden Reste trägt: $C_1$-$C_6$-Alkoxyimino, $C_3$-$C_6$-Alkenyloxyimino, $C_3$-$C_6$-Halogenalkenyloxyimino oder Benzyloxyimino;

eine $C_3$-$C_6$-Alkenyl- oder eine $C_3$-$C_6$-Alkinylgruppe, wobei diese Gruppen ihrerseits ein bis fünf Halogenatome tragen können.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Derivates I gemäß Anspruch 1 behandelt.

6. Mittel zur Beeinflussung des Pflanzenwuchses, enthaltend ein Benzaldoximetherderivat der Formel I gemäß Anspruch 1 und übliche inerte Zusatzstoffe.

7. Verfahren zur Regulierung des Pflanzenwuchses, dadurch gekennzeichnet, daß man eine regulativ wirksame Menge eines Benzaldoximetherderivates der Formel I gemäß Anspruch 1 auf die Samen, die

Pflanzen und/oder deren Lebensraum einwirken läßt.

8. Verfahren zur Herstellung von Verbindungen der Formel IV,

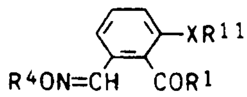

IV

in der $R^{11}$ für Wasserstoff, Acetyl oder Methyl steht, dadurch gekennzeichnet, daß man die entsprechenden Aldehyde

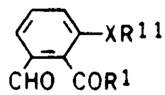

mit den Aminen $R^4ONH_2$ umsetzt.

Europäisches Patentamt

# EUROPÄISCHER RECHERCHENBERICHT

Nummer der Anmeldung

**EP 91 11 5908**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | EP-A-0 287 079 (KUMIAI CHEMICAL INDUSTRY)<br>* Seite 5 - Seite 12; Ansprüche * *<br>- - - | 1,2,7-10 | C 07 D 239/60<br>C 07 D 239/52<br>C 07 D 251/30<br>C 07 D 403/12<br>C 07 C 251/32<br>A 01 N 43/54<br>A 01 N 43/66 |
| A | EP-A-0 346 789 (BASF)<br>* Seite 26 - Seite 29 * *<br>- - - | 1,2,7-10 | |
| X | FR-A-2 186 256 (ENDO LABORATORIES)<br>* Seite 7 - Seite 13 * *<br>- - - | 5 | |
| P,X | EP-A-0 435 170 (KUMIAI CHEMICAL INDUSTRY)<br>* INSGESAMT * *<br>- - - - - | 1-10 | |

| RECHERCHIERTE SACHGEBIETE (Int. Cl.5) |
|---|
| C 07 D |

Der vorliegende Recherchenbericht wurde für alle Patentansprüche erstellt

| Recherchenort | Abschlußdatum der Recherche | Prüfer |
|---|---|---|
| Den Haag | 30 Januar 92 | FRANCOIS J.C.L. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument